# EUROPEAN PATENT SPECIFICATION

(11) **EP 1 895 984 B1**
(45) Date of publication and mention of the grant of the patent: **03.03.2010**
(21) Application number: 06772091.2
(22) Date of filing: 05.06.2006
(51) Int. Cl.: A61K 9/14, A61K 31/506

(54) **NANOPARTICULATE IMATINIB MESYLATE FORMULATIONS**
NANOTEILCHENFÖRMIGE IMATINIB-MESYLAT-FORMULIERUNGEN
FORMULATIONS D'IMATINIB MESYLATE NANOPARTICULAIRES

(30) Priority: 03.06.2005 US 687146 P
(43) Date of publication of application: 12.03.2008
(73) Proprietor: Elan Pharma International Limited, Dublin 2 (IE)
(72) Inventor: JENKINS, Scott, Downingtown, PA 19335 (US); LIVERSIDGE, Gary, G., Westchester, PA 19380 (US)
(74) Representative: Pohlman, Sandra M.
(86) International application number: PCT/US2006/021657
(87) International publication number: WO 2006/133046

(56) References cited:
- WO-A-2006/054314
- WO-A-2006/110811
- US-A1- 2005 042 177

## Description

### FIELD

The invention relates generally to compounds and compositions useful in the treatment of chronic myeloid leukemia, gastrointestinal stromal tumors and related diseases. More specifically, the invention relates to nanoparticulate imatinib mesylate compositions. The nanoparticulate imatinib mesylate compositions have an effective average particle size of less than about 2000 mm.

### BACKGROUND

### A. Background Regarding Imatinib mesylate

Imatinib mesylate, chemically known as 4-[(4-Methyl-1-piperazinyl)methyl]-N-[4-methyl-3-[[4-(3-pyridinyl)-2-pyrimidinyl]amino]-phenyl]benzamide methanesulfonate, has a molecular formula of C₂₉H₃₁N₇O·CH₄SO₃ , and a molecular weight of 589.7.

Imatinib mesylate has the chemical structure shown below:

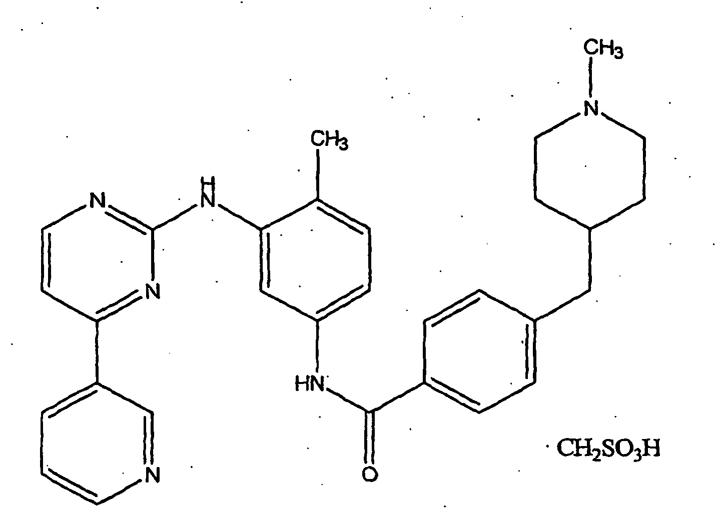

Imatinib mesylate is a white to off-white to brownish or yellowish tinged crystalline powder. Imatinib mesylate is soluble in aqueous buffers ≤ pH 5.5 and slightly soluble to insoluble in neutral to alkaline aqueous buffers. In non-aqueous solvents, imatinib mesylate is freely soluble to very slightly soluble in dimethyl sulfoxide, methanol and ethanol, but is insoluble in n-octanol, acetone and acetonitrile.

Imatinib mesylate is commercially available under the trade name Gleevec® as film-coated tablets, manufactured by Novartis Pharma Stein AG (Stein, Switzerland), and distributed by Novartis Pharmaceuticals Corporation (East Hanover, New Jersey). Gleevec® is available in strengths containing imatinib mesylate in amounts equivalent to 100 mg or 400 mg of imatinib free base. Gleevec® contains inactive ingredients that include colloidal silicon dioxide; crospovidone; hydroxypropyl methylcellulose; magnesium stearate; and microcrystalline cellulose with tablet coatings having ferric oxide, red; ferric oxide, yellow; hydroxypropyl methylcellulose; polyethylene glycol and talc.

Imatinib mesylate is indicated for the treatment of Philadelphia chromosome positive chronic myeloid leukemia (CML) and Kit (CD117) positive unresectable and/or metastatic malignant gastrointestinal stromal tumors (GIST).

Gleevec® is generally prescribed in dosages of 400 mg/day for adult patients in chronic phase CML and 600 mg/day for adult patients in accelerated phase or blast crisis. Additionally Gleevec® is recommenced at dosages of 400 mg/day or 600 mg/day for adult patients with unresectable and/or metastatic, malignant GIST. Gleevec® is generally prescribed to be administered orally, with a meal and a large glass of water, with doses of 400 mg or 600 mg administered once daily, and dosages of 800 mg administered as 400 mg twice a day.

Imatinib mesylate compounds have been disclosed, for example, in United States Patent No. 5,521,184 to Zimmermann for "Pyrimidine Derivatives and Processes for the Preparation Thereof" and United States Patent Application No. 2004/0127571 to Bhalla et al. for "Method of Treating Leukemia with a Combination of Suberoylanilide Hydromaxic Acid and Imatinib Mesylate".

Imatinib mesylate has high therapeutic value in the treatment of chronic myeloid leukemia, gastrointestinal stromal tumors, and related diseases. However, because conventional, non-nanoparticulate imatinib mesylate tablets are only very slightly soluble in water at 37°C, the dissolution of conventional imatinib mesylate tablets is reduced in the fasting state as compared to the fed state. Thus, imatinib mesylate has limited bioavailability in the fasting state as compared to the fed state, which limits the therapeutic outcome for all treatments requiring imatinib mesylate.

### B. Background Regarding Nanoparticalate Active Agent Compositions

Nanoparticulate active agent compositions, first described in U.S. Patent No. 5,145,684 ("the '684 patent"), are particles consisting of a poorly soluble therapeutic or diagnostic agent having adsorbed onto the surface thereof a non-crosslinked surface stabilizer. The '684 patent does not describe nanoparticulate compositions of imatinib mesylate.

Methods of making nanoparticulate active agent compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate active agent compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble NonMagnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,44.0 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate UNSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging, X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulixed Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill," and 6,432,381 for "Methods for Targeting Drug Delivery to the Upper and/or Lower Gastrointestinal Tract," 6,592,903 for "Nanoparticulate Dispersions Comprising a Synergistic Combination of a Polymeric Surface Stabilizer and Dioctyl Sodium Sulfosuccinate," 6,582,285 for "Apparatus for sanitary wet milling;" 6,656,504 for Nanoparticulate Compositions Comprising Amorphous Cyclosporine;" 6,742,734 for "System and Method for Milling Materials;" 6,745,962 for "Small Scale Mill and Method Thereof;" 6,811,767 for "Liquid droplet aerosols of nanoparticulate drugs;" and 6,908,626 for "Compositions having a combination of immediate release and controlled release characteristics;" 6,969,529 for "Nanoparticulate compositions comprising copolymers of vinyl pyrrolidone and vinyl acetate as surface stabilizers;" 6,976,647 for "System and Method for Milling Material". In addition, U.S. Patent Publication No. 20020012675. A1, for "Controlled Release Nanoparticulate Compositions;" U.S. Patent Publication No. 20050276974 for "Nanoparticulate Fibrate Formulations;" U.S. Patent Publication No. 20050238725 for "Nanoparticulate compositions having a peptide as a surface stabilizer," U.S. Patent Publication No. 20050233001 for "Nanoparticulate megestrol formulations;" U.S. Patent Publication No. 20050147664 for "Compositions comprising antibodies and methods of using the same for targeting nanoparticulate active agent delivery," U.S. Patent Publication No. 20050063913 for "Novel metaxalone compositions;" U.S. Patent Publication No. 20050042177 for "Novel compositions of sildenafil free base;" U.S. Patent Publication No. 20050031691 for "Gel stabilized nanoparticulate active agent compositions;" U.S. Patent Publication No. 20050019412 for " Novel glipizide compositions;",U.S. Patent Publication No. 20050004049 for "Novel griseofulvin compositions;" U.S. Patent Publication No. 20040258758 for "Nanoparticulate topiramate formulations;" U.S. Patent Publication No. 20040258757 for " Liquid dosage compositions of stable nanoparticulate active agents;" U.S. Patent Publication No. 20040229038 for "Nanoparticulate meloxicam formulations;" U.S. Patent Publication No. 20040208833 for "Novel fluticasone formulations;" U.S. Patent Publication No. 20040195413 for " Compositions and method for milling materials;" U.S. Patent Publication No. 20040156895 for "Solid dosage forms comprising pullulan;" U.S. Patent Publication No. U.S. Patent Publication No. U.S. Patent Publication No. 20040156872 for "Novel nimesulide compositions;" U.S. Patent Publication No. 20044141925 for "Novel triamcinolone compositions;" U.S. Patent Publication No. 20040115134 for "Novel nifedipine compositions;" U.S. Patent Publication No. 20040105889 for "Low viscosity liquid dosage forms;" U.S. Patent Publication No. 20040105778 for "Gamma irradiation of solid nanoparticulate active agents;" U.S. Patent Publication No. 20040101566 for "Novel benzoyl peroxide compositions;" U.S. Patent Publication No. 20040057905 for "Nanoparticulate beclomethasone dipropionate compositions;" U.S. Patent Publication No. 20040033267 for "Nanoparticulate compositions of angiogenesis inhibitors;" U.S. Patent Publication No. 20040033202 for "Nanoparticulate sterol formulations and novel sterol combinations;" U.S. Patent Publication No. 20040018242 for "Nanoparticulate nystatin formulations;" U.S. Patent Publication No. 20040015134 for "Drug delivery systems and methods;" U.S. Patent Publication No. 20030232796 for "Nanoparticulate polycosanol formulations & novel polycosanol combinations;" U.S. Patent Publication No. 20030215502 for "Fast dissolving dosage forms having reduced friability;" U.S. Patent Publication No. 20030185869 for "Nanoparticulate compositions having lysozyme as a surface stabilizer;" U.S. Patent Publication No. 20030181411 for "Nanoparticulate compositions of mitogen-activated protein (MAP) kinase inhibitors;" U.S. Patent Publication No. 20030137067 for "Compositions having a combination of immediate release and controlled release characteristics;" U.S. Patent Publication No. 20030108616 for "Nanoparticulate compositions comprising copolymers of vinyl pyrrolidone and vinyl acetate as surface stabilizers;" U.S. Patent Publication No. 20030095928 for "Nanoparkiculate insulin;" U.S. Patent Publication No. 20030087308 for "Method for high through put screening using a small scale mill or microfluidics;" U.S. Patent Publication No. 20030023203 for "Drug delivery systems & methods;" U.S. Patent Publication No. 20020179758 for "System and method for milling material; and U.S. Patent Publication No. 20010053664 for "Apparatus for sanitary wet milling," describe nanoparticulate active agent compositions.

Surface modified nanoparticles and compositions thereof useful for treating cancer and other neoplastic diseases have been described, for example, in U.S. Patent Nos. 5,399,363 and 5,494,683, both for "Surface Modified Anticancer Nanoparticles".

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

There is a need in the art for imatinib mesylate formulations which overcome the fed/fasted absorption variability, along with other problems, observed with conventional imatinib mesylate dosage forms, The present invention, which overcome such problems, relates to a nanoparticulate composition comprising imatinib mesylate, or a salt thereof for the treatment of chronic myeloid leukemia, gastrointestinal stromal tumors and related diseases.

### SUMMARY

The compositions disclosed herein typically include nanoparticulate imatinib mesylate, or a salt thereof having an effective average particle size of less than about 2000 nm and at least one surface stabilizer. The surface stabilizer is typically adsorbed on or associated with the surface of the nanoparticulate imatinib mesylate particles. Optionally, the compositions may include a pharmaceutically acceptable carrier and any suitable excipients.

The nanoparticulate compositions of imatinib mesylate, or a salt thereof, disclosed herein may be effective in the treatment of a number of disease or conditions, including but not limited to chronic myeloid leukemia, gastrointestinal stromal tumors and related diseases.

A preferred dosage form of the invention is a solid dosage form, although any pharmaceutically acceptable dosage form can be utilized.

Another aspect of the invention is directed to pharmaceutical composition comprising particles of a nanoparticulate imatinib mesylate, or a salt thereof, at least one surface stabilizer, and a pharmaceutically acceptable carrier, as well as any desired excipients.

One embodiment of the invention encompasses a nanoparticulate imatinib mesylate composition, wherein the pharmacokinetic profile of the nanoparticulate imatinib mesylate is not affected by the fed or fasted state of a subject ingesting the composition.

In yet another embodiment, the invention encompasses a nanoparticulate imatinib mesylate composition, wherein administration of the composition to a subject in a fasted state is bioequivalent to administration of the composition to a subject in a fed state.

Another embodiment of the invention is directed to nanoparticulate imatinib mesylate compositions comprising one or more additional compounds useful in the treatment of chronic myeloid leukemia, gastrointestinal stromal tumors, and related disease.

This invention further discloses a method of making the nanoparticulate imatinib mesylate composition. Such a method comprises contacting the nanoparticulate imatinib mesylate, or a salt thereof, with at least one surface stabilizer for a time and under conditions sufficient to provide a nanoparticulate imatinib mesylate composition having an effective average particle size of less than about 2000 nm. The one or more surface stabilizers can be contacted with a nanoparticulate imatinib mesylate, either before, during, or after size reduction of the imatinib mesylate particle.

The present invention is also directed to methods of treatment including but not limited to, the treatment of chronic myeloid leukemia, gastrointestinal stromal tumors and related diseases, using the novel nanoparticulate imatinib mesylate compositions disclosed herein, Such methods comprise administering to a subject a therapeutically effective amount of a nanoparticulatc imatinib mesylate, or a salt thereof. Other methods of treatment using the nanoparticulate compositions of the invention are known to those of skill in the art.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPTION OF THE PREFERRED EMBODIMENTS

The present invention is directed to nanoparticulate compositions comprising an imatinib mesylate, or a salt thereof. The compositions comprise an imatinib mesylate, or a salt thereof, and preferably at least one surface stabilizer adsorbed on or associated with the surface of the drug. The imatinib mesylate, or a salt thereof, particles have an effective average particle size of less than about 2000 nm.

Advantages of the nanoparticulate imatinib mesylate compositions of the invention as compared to a conventional, non-nanoparticulate composition of the same imatinib mesylate formulation, include, but are not limited to: (1) smaller tablet size or other solid dosage form size; (2) smaller doses of drug required to obtain the same pharmacological effect; (3) increased bioavailability; (4) substantially similar pharmacokinetic profiles of the imatinib mesylate compositions when administered in the fed versus the fasted state; (5) bioequivalency of the imatinib mesylate compositions; (6) an increased rate of dissolution for the imatinib mesylate compositions; (7) the imatinib mesylate, nanoparticles of the present invention redisperse upon addition thereof to a solution; and (8) the imatinib mesylate compositions can be used in conjunction with other active agents useful in the treatment of chronic myeloid leukemia, gastrointestinal stromal tumors and related diseases.

The present invention also includes nanoparticulate imatinib mesylate compositions, or a salt thereof, together with one or more non-toxic physiologically acceptable carriers, adjuvants, or vehicles, collectively referred to as carriers. The compositions can be formulated for parenteral injection (*e.g*.; intravenous, intramuscular, or subcutaneous), oral administration in solid, liquid, or aerosol form, vaginal, nasal, rectal, ocular, local (powders, ointments, or drops), buccal, intracisternal, intraperitoneal, or topical administration, and the like.

A preferred dosage form of the invention is a solid dosage form, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage for, pulsatile release dosage form, mixed immediate release and controlled release dosage form, or a combination thereof. A solid dose tablet formulation is preferred.

The present invention is described herein using several definitions, as set forth below and throughout the application.

The term "effective average particle size of less than about 2000 nm," as used herein, means that at least about 50% of the nanoparticulate imatinib mesylate particles have a size of less than about 2000 nm, by weight (or by other suitable measurement technique, such as by number, volume, etc.) when measured by, for example, sedimentation flow fractionation, photon correlation spectroscopy, light scattering, disk centrifugation, and other techniques known to those of skill in the art.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable imatinib mesylate nanoparticulate particles, "stable" connotes, but is not limited to one or more of the following parameters: (1) the particles do not appreciably flocculate or agglomerate due to interparticle attractive forces or otherwise significantly increase in particle size over time; (2) that the physical structure of the particles is not altered over time, such as by conversion from an amorphous phase to a crystalline phase; (3) that the particles are chemically stable; and/or (4) where the imatinib mesylate has not been subject to a heating step at or above the melting point of the imatinib mesylate in the preparation of the nanoparticles of the present invention.

The term "conventional" or "non-nanoparticulate active agent" shall mean an active agent which is solubilized or which has an effective average particle size of greater than about 2000 nm. Nanoparticulate active agents as defined herein have an effective average particle size of less than about 2000 nm.

The phrase "poorly water soluble drugs" as used herein refers to those drugs that have a solubility in water of less than about 30 mg/ml, less than about 20 mg/ml, less than about 10 mg/ml, or less than about 1 mg/ml.

As used herein, the phrase "therapeutically effective amount" shall mean that drug dosage that provides the specific pharmacological response for which the drug is Administered in a significant number of subjects in need of such treatment. It is emphasized that a therapeutically effective amount of a drug that is administered to a particular subject in a particular instance will not always be effective in treating the conditions/diseases described herein, even though such dosage is deemed to be a therapeutically effective amount by those of skill in the art.

### A. Preferred Characteristics of the Nanoparticulate Imatinib Mesylate Compositions of the Invention

### 1. Increases Bioavailability

The nanoparticulate imatinib mesylate, or a salt thereof, formulations of the invention are proposed to exhibit increased bioavailability, and require smaller doses as compared to prior conventional imatinib mesylate formulations.

In one embodiment of the invention, the nanoparticulate imatinib mesylate composition, upon administration to a mammal, produces therapeutic results at a dosage which is less than that of a non-nanoparticulate dosage form of the same imatinib mesylate composition.

### 2. Improved Pk Profiles

The invention also preferably provides compositions comprising nanoparticulate imatinib mesylate, or a salt thereof, having a desirable pharmacokinetic profile when administered to mammalian subjects. The desirable pharmacokinetic profile of the compositions comprising imatinib mesylate, or a salt thereof, preferably includes, but is not limited to: (1) a Cₘₐₓ for imatinib mesylate, when assayed in the plasma of a mammalian subject following administration, that is preferably greater than the Cₘₐₓ for a non-nanoparticulate formulation of the same imatinib mesylate, administered at the same dosage; and/or (2) an AUC for imatinib mesylate, when assayed in the plasma of a mammalian subject following administration, that is preferably greater than the AUC for a non-nanoparticulate formulation of the same imatinib mesylate, administered at the same dosage; and/or (3) a Tₘₐₓ for imatinib mesylate, when assayed in the plasma of a mammalian subject following administration, that is preferably less than the Tₘₐₓ for a non-nanoparticulate formulation of the same imatinib mesylate, administered at the same dosage.

In one embodiment, a composition comprising a nanoparliculate imatinib mesylate exhibits in comparative pharmacokinetic testing with a non-nanoparticulate formulation of the same imatinib mesylate, administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate imatinib mesylate formulation.

In another embodiment, the composition comprising a nanoparticulate imatinib mesylate exhibits in comparative pharmacokinctic testing with a non-Nanoparticulate formulation of the same imatinib mesylate, administered at the same dosage, a Cₘₐₓ which is at least about 50%, at least about 100%, at least about 200%, at least about 304%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate imatinib mesylate formulation.

In yet another embodiment, the composition comprising a nanoparticulate imatinib mesylate exhibits in comparative pharmacokinetic testing with a non-nanoparticulate formulation of the same imatinib mesylate, administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at leat about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate imatinib mesylate formulation.

In one embodiment of the invention, the Tₘₐₓ of imatinib mesylate, when assayed in the plasma of the manmalian subject, is less than about 6 to about 8 hours. In other embodiments of the invention, the Tₘₐₓ of imatinib mesylate is less than about 6 hours, less than about 5 hours, less than about 4 hours, less than about 3 hours, less than about 2 hours, less than about 1 hour, or less than about 30 minutes after administration.

The desirable pharmacokinetic profile, as used herein, is the pharmacokinetic profile measured after the initial dose of imatinib mesylate or a salt thereof. The compositions can be formulated in any way as described herein and as known to those of skill in the art.

### 3. The Pharmacokinetic Profiles of the Imatinib Mesylate Compositions of the Invention are not Affected by the Fed or Fasted State of the Subject Ingesting the Compositions

The invention encompasses imatinib mesylate compositions wherein the pharmacokinetic profile of imatinib mesylate is not substantially affected by the fed or fasted state of a subject ingesting the composition. This means that there is no substantial difference in the quantity of drug absorbed or the rate of drug absorption when the nanoparticulate imatinib mesylate compositions are administered in the fed versus the fasted state.

For conventional imatinib mesylate formulations, *i.e.*, GLEEVEC®, the absorption of imatinib mesylate is increased when administered with food. This difference in absorption observed with conventional imatinib mesylate formulations is undesirable. The imatinib mesylate formulations of the invention overcome this problem, as the imatinib mesylate formulations reduce or preferably substantially eliminate significantly different absorption levels when administered under fed as compared to fasting conditions.

Benefits of a dosage form which substantially eliminates the effect of food include an increase in subject convenience, thereby increasing subject compliance, as the subject does not need to ensure that they are taking a dose either with or without food. This is significant, as with poor subject compliance an increase in the medical condition for which the drug is being prescribed may be observed.

### 4. Bioequivalency of Imatinib Mesylate Compositions of the Invention When Administered in the Fed Versus the Fasted State

The invention also provides a nanoparticulate imatinib mesylate composition in which the administration of the composition to a subject in a fasted state is bioequivalent to administration of the composition to a subject in a fed state.

The difference in absorption of the imatinib mesylate compositions of the invention, when administered in the fed versus the fasted state, preferably is less than about 60%, less than about 55%, less than about 50%, less than about 45%, less than about 40%, less than about 35%, less than about 30%, less than about 25%, less than about 20%, less than about 15%, less than about 10%, less than about 5%, or less than about 3 %.

In one embodiment of the invention, the invention encompasses compositions comprising at least one nanoparticulate imatinib mesylate composition, wherein administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% CI for AUC must be between 0.80 to 1.25 and the 90% CI for Cₘₐₓ must between 0.70 to 1.43.

### 5. Dissolution Profiles of the Imatinib Mesylate Compositions of the Invention

The nanoparticulate imatinib mesylate compositions, or a salt thereof, of the invention are proposed to have unexpectedly dramatic dissolution profiles. Rapid dissolution of an administered active agent is preferable, as faster dissolution generally leads to faster onset of action and greater bioavailability. To improve the dissolution profile and bioavailability of the imatinib mesylate it would be useful to increase the drug's dissolution so that it could attain a level close to 100%.

The imatinib mesylate compositions of the invention preferably have a dissolution profile in which within about 5 minutes at least about 20% of the composition is dissolved. In other embodiments of the invention, at least about 30% or at least about 40% of the imatinib mesylate composition is dissolved within about 5 minutes. In yet other embodiments of the invention, at least about 40%, at least about 50%, at least about 60%, at least about 70%, or at least about 80% of the imatinib mesylate composition is dissolved within about 10 minutes. Finally, in another embodiment of the invention, preferably at least about 70%, at least about 80%, at least about 90%, or at least about 100% of the imatinib mesylate composition is dissolved within about 20 minutes.

Dissolution is preferably measured in a medium which is discriminating. Such a dissolution medium will produce two very different dissolution curves for two products having very different dissolution profiles in gastric juices; i.e., the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### 6. Redispersibility Profiles of the Imatinib Mesylate Compositions of the Invention

An additional feature of the imatinib mesylate, or a salt thereof, compositions of the invention is that the compositions redisperse such that the effective average particle size of the redispersed imatinib mesylate particles is less than about 2 microns. This is significant, as if upon administration the imatinib mesylate compositions of the invention did not redisperse to a substantially nanoparticulate size, then the dosage form may lose the benefits afforded by formulating the imatinib mesylate into a nanoparticulate particle size.

This is because nanoparticulate active agent compositions benefit from the small particle size of the active agent; if the active agent does not redisperse into the small particle sizes upon administration, then "clumps" or agglomerated active agent particles are formed, owing to the extremely high surface free energy of the nanoparticulate system and the thermodynamic driving force to achieve an overall reduction in free energy. With the formation of such agglomerated particles, the bioavailability of the dosage form may fall.

Moreover, the nanoparticulate imatinib mesylate compositions of the invention exhibit dramatic redispersion of the nanoparticulate imatinib mesylate composition particles upon administration to a mammal, such as a human or animal, as demonstrated by reconstitution/redispersion in a biorelevant aqueous media such that the effective average particle size of the redispersed imatinib mesylate composition particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. See e.g., Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (i.e., weak acids and corresponding salts of that acid), monoprotic and polyprotic electrolytes, etc.

Representative electrolyte solutions can be, but are not limited to, HCl solutions, ranging in concentration from about 0.001 to about 0.1 N, and NaCl solutions, ranging in concentration from about 0.001 to about 0.1 M, and mixtures thereof. For example, electrolyte solutions can be, but are not limited to, about 0.1 N HCl or less, about 0.01 N HCl or less, about 0.001 NHCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 N HCl, 0.01 N HCl, and 0.1 N HCl correspond to pH 3, pH 2, and pH 1, respectively. Thus, a 0.01 N HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed particles of imatinib mesylate, or a salt thereof, (redispersed in water, a biorelevant media, or any other suitable media) have an effective average particle size of less than about less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than .about 650 nm, less than about 600 nm, less than about 550 nm, less than about 500 nm, less than about 450, less than about 400 nm, less than about 350 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods. Such methods suitable for measuring effective average particle size are known to a person of ordinary skill in the art.

Redispersibility can be tested using any suitable means known in the art. See e.g., the example sections of U.S. Patent No. 6,375,986 for "Solid Dose Nanoparticulate Compositions Comprising a Sydergistic Combination of a Polymeric Surface Stabilizer and Dioctyl Sodium Sulfosuccinate."

### 7. Imatinib Mesylate Compositions Used in Conjunction with Other Active Agents

The imatinib mesylate, or a salt thereof, compositions of the invention can additionally comprise one or more compounds useful in the treatment of chronic myeloid leukemia, gastrointestinal stromal tumors and related diseases, or the imatinib mesylate compositions can be administered in conjunction with such a compounds Examples of such compounds include, but are not limited to, anti-cancer agents such as mitotic inhibitors, alkylating agents, anti-metabolites, intercalating antibiotics, growth factor inhibitors, cell cycle inhibitors, enzymes, topoisomerase inhibitors, biological response modifiers, anti-hormones, and anti-androgens. For example, additional compounds may include gefitinib, pertuzamib, paclitaxel, cisplatin, carboplatin, gemcitabine, bevacizumab, temoxolomide, sutent, leflunomide, docetaxel, imatinib, laptinib, canertinib, doxorubincin, vatalanib, sorafenib, leucovorin, capecitabine, cetixuimab, and combinations thereof.

### B. Nanoparticulate Imatinib Mesylate Compositions

The invention provides compositions comprising particles of imatinib mesylate, or a salt thereof, and at least one surface stabilizer. The surface stabilizers preferably are adsorbed on, or associated with, the surface of the imatinib mesylate particles. Surface stabilizers especially useful herein preferably physically adhere on, or associate with, the surface of the nanoparticulate imatinib mesylate particles, but do not chemically react with the imatinib mesylate particles or itself. Individually adsorbed molecules of the surface stabilizer are essentially free of intermolecular cross-linkages.

The present invention also includes imatinib mesylate, or a salt thereof, compositions together with one or more non-toxic physiologically acceptable carriers, adjuvants, or vehicles, collectively referred to as carriers. The compositions can be formulated for parenteral injection (e.g., intravenous, intramuscular, or subcutaneous), oral administration in solid, liquid, or aerosol form, vaginal, nasal, rectal, ocular, local (powders, ointments or drops), buccal, intracisternal, intraperitoneal, or topical administration, and the like.

### 1. Imatinib Mesylate

The compositions of the invention comprise imatinib mesylate or a salt thereof. The particles of imatinib mesylate, or a salt thereof can be in a crystalline phase, a semi-crystalline phase, an amorphous phase, a semi-amorphous phase, or a combination thereof.

Imatinib mesylate has the molecular formula (formula I):

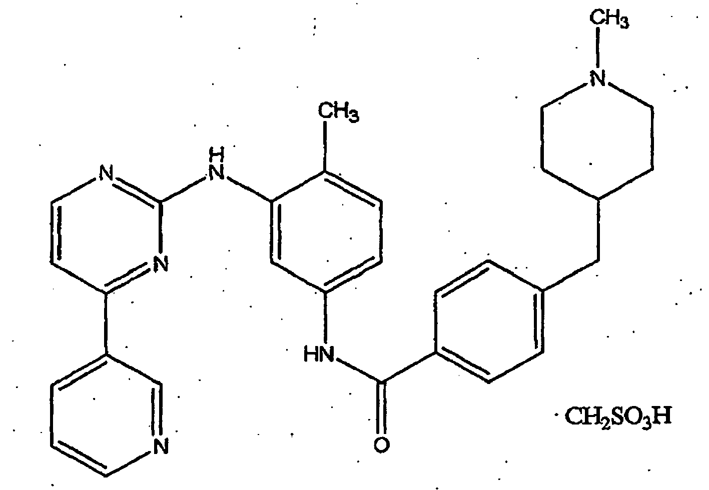

### 2. Surface Stabilizers

Combinations of more than one surface stabilizer can be used in the invention. Useful surface stabilizers which can be employed in the invention include, but are not limited to, known organic and inorganic pharmaceutical excipients. Such excipients include various polymers; low molecular weight oligomers, natural products, and surfactants. Exemplary surface stabilizers include nonionic, ionic, anionic, cationic, and zwitterionic compounds or surfactants.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.*, macrogol ethers such as cetomacrogol 1000), polyoxyethylene castor oil derivatives, polyoxyethylene sorbitan fatty acid esters (*e.g*., the commercially available Tween^{®} products such as *e.g.*, Tween^{®} 20 and Tween^{®} 80 (ICI Speciality Chemicals)); polyethylene glycols (*e.g.*, Carbowax^{®} 3550 and 934 (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronic^{®} F68 and F108, which are block copolymers of ethylene oxide and propylene oxide); poloxamines (*e.g.*, Tetronic^{®} 908, also know as Poloxamine^{®} 908, which is a tetrafunctional block copolymer derived from sequential addition of propylene oxide and ethylene oxide to ethylenediamine (BASF Wyandotte Corporation, Parsippany, N.J.)); Tetronic^{®} 1508 (T-1508) (BASF Wyandotte Corporation), Triton^{®} X-200, which is an alkyl aryl polyether sulfonate (Rohm and Haas); Crodesta^{®} F-110, which is a mixture of sucrose stearate and sucrose,distearate (Croda Inc.); p-isononylphenoxypoly-(glycidol), also known as Olin^{®}-IOG or Surfactant^{®} 10-G (Olin Chemicals, Stamford, CT); Crodestas^{®} SL-40 (Croda, Inc.); and SA9OHCO which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, such as Plasdone^{®} S630 and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimcthylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL™ and ALKAQUAT™(Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such as benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, bezalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000).

The imatinib mesylate composition and surface stabilizer may be present in the pharmaceutical compositions disclosed herein at any suitable ratio (w/w). For example, in some embodiments the pharmaceutical compositions include the imatinib mesylate composition and the surface stabilizer at a ratio of about 20:1,15:1,10:1, 8:1, 7:1,6:1, 5:1, 4:1, 3:1, 2:1 (w/w), or any range defined by said ratios (for example, but not limited to about 20:1- 2:1, about 10:1 - 4:1, and about 8:1 - 5:1).

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Imatinib Mesylate Particle Size

The compositions of the invention comprise nanoparticulate imatinib mesylate, or a salt thereof particles which have an effective average particle size of less than about 2000 nm (*i.e.,* 2 microns), less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 mn, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the imatinib mesylate particles have a particle size of less than the affective average, by weight (or by other suitable measurement technique, such as by volume, number etc.), *i.e.,* less than about 2000 nm,1900 nm, 1800 nm, *etc.,* when measured by the above-noted techniques. In other embodiments of the invention, at least about 60%, at least about 70%, at least about 80%, at least about 90%, at least about 95%, or at least about 99% of the imatinib mesylate particles have a particle size of less than the effective average, *i.e*., less than about 2000 nm, 1900 nm, 1800 nm, 1700 nm, *etc*.

In the present invention, the value for D50 of a nanoparticulate imatinib mesylate composition is the particle size below which 50% of the imatinib mesylate particles fall, by weight (or by other suitable measurement technique, such as by volume, number etc.). Similarly, D90 is the particle size below which 90% of the imatinib mesylate particles fall, by weight (or by other suitable measurement technique, such as by volume, number etc.).

### 5. Concentration of Imatinib Mesylate and Surface Stabilizers

The relative amounts of imatinib mesylate, or a salt thereof, and one or more surface stabilizers can vary widely. The optimal amount of the individual components can depend, for example, upon the particular imatinib mesylate selected, the hydrophilic lipophilic balance (HLB), melting point, and the surface tension of water solutions of the stabilizer, *etc*.

The concentration of the imatinib mesylate can vary from about 99.5% to about 0.001 %, from about 95% to about 0.1%, or from about 90% to about 0.5%, by weight, based on the total combined dry weight of the imatinib mesylate and at least one surface stabilizer, not including other excipients.

The concentration of the at least one surface stabilizer can vary from about 0.5% to about 99.999%, from about 5.0% to about 99.9%, or from about 10% to about 99.5%, by weight, based on the total combined dry weight of the imatinib mesylate and at least one surface stabilizer, not including other excipients.

### 6. Exemplary Nanoparticulate Imatinib Mesylate Tablet Formulations

Several exemplary imatinib mesylate tablet formulations are given below. These examples are not intended to limit the claims in any respect, but rather to provide exemplary tablet formulations of imatinib mesylate which can be utilized in the methods of the invention. Such exemplary tablets can also comprise a coating agent.

| **Exemplary Nanoparticulate Imatinib Mesylate Tablet Formulation #1** | |
|---|---|
| Component | g/Kg |
| Imatinib Mesylate | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF - | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

| **Exemplary Nanoparticulate Imatinib Mesylate Tablet Formulation #2** | |
|---|---|
| Component | g/Kg |
| Imatinib Mesylate | about 100 to about 300 |
| Hypromellose, USP | about 30 to about 50 |
| Docusate Sodium, USP | about 0.5 to about 10 |
| Sucrose, NF | about 100 to about 300 |
| Sodium Lauryl Sulfate, NF | about 1 to about 30 |
| Lactose Monohydrate, NF | about 100 to about 300 |
| Silicified Microcrystalline Cellulose | about 50 to about 200 |
| Crospovidone, NF | about 50 to about 200 |
| Magnesium Stearate, NF | about 0.5 to about 5 |

| **Exemplary Nanoparticulate Imatinib Mesylate Tablet Formulation #3** | |
|---|---|
| Component | g/Kg |
| Imatinib Mesylate | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

| **Exemplary Nanoparticulate Imatinib Mesylate Tablet Formulation #4** | |
|---|---|
| Component | g/Kg |
| Imatinib Mesylate | abouf 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose, | about 129 to about 134 |
| Crospovidone, NF | about 112 to about 118 |
| Magnesium Stearate, NF | about 0.5 to about 3 |

### C. Methods of Making "Nanoparticulate Imatinib Mesylate Compositions

The nanoparticulate imatinib mesylate, or a salt thereof, compositions can be made using, for example, milling, homogenization, precipitation, cryogenic, or template emulsion techniques. Exemplary methods of making nanoparticulate active agent compositions are described in the '684 patent. Methods of making nanoparticulate active agent compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Natioparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-ray Contrast Compositions Containing Nanoparticles;" U.S. Patent No. 5,534,270 for "Method of Preparing Stable Drug Narioparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation".

The resultant nanoparticulate imatinib mesylate compositions or dispersions can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, *etc*.

### 1. Milling to Obtain Nanoparticutate Imatinib Mesylate Dispersions

Milling an Imatinib mesylate, or a salt thereof, to obtain a nanoparticulate dispersion comprises dispersing the imatinib mesylate particles in a liquid dispersion medium in which the imatinib mesylate is poorly soluble, followed by applying mechanical means in the presence of grinding media to reduce the particle size of the imatinib mesylate to the desired effective average particle size. The dispersion medium can be, for example, water, safflower oil, ethanol, t-butanol, glycerin, polyethylene glycol (PEG), hexane, or glycol. A preferred dispersion medium is water.

The imatinib mesylate particles can be reduced in size in the presence of at least one surface stabilizer. Alternatively, imatinib mesylate particles can be contacted with one or more surface stabilizers after attrition. Other compounds, such as a diluent, can be added to the imatinib mesylate/surface stabilizer composition during the size reduction process. Dispersions can be manufactured continuously or in a batch mode.

### 2. Precipitation to Obtain Nanoparticulate Imatinib Mesylate Compositions

Another method of forming the desired nanoparticulate imatinib mesylate, or a salt thereof, composition is by microprecipitation. This is a method of preparing stable dispersions of poorly soluble active agents in the presence of one or more surface stabilizers and one or more.colloid stability enhancing surface active agents free of any trace toxic solvents or solubilized heavy metal impurities. Such a method comprises, for example: (1) dissolving the imatinib mesylate in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nanopardculate Imatinib Mesylate Compositions

Exemplary homogenization methods of preparing active agent nanoparticulate compositions are described in U.S. Patent No. 5,510,118, for "Process of Preparing Therapeutic Compositions Containing Nanoparticles." Such a method comprises dispersing particles of an imatinib mesyiate, or a salt thereof in a liquid dispersion medium, followed by subjecting the dispersion to homogenization to reduce the particle size of an imatinib mesylate to the desired effective average particle size. The imatinib mesylate particles can be reduced in size in the presence of at least one surface stabilizer. Alternatively, the imatinib mesylate particles can be contacted with one or more surface stabilizers either before or after attrition. Other compounds, such as a diluent, can be added to the imatinib mesylate/surface stabilizer composition either before, during, or after the size reduction process. Dispersions can be manufactured continuously or in a batch mode.

### 4. Cryogenic Methodologies to Obtain Nanoparticulate Imatinib Mesylate Compositions

Another method of forming the desired nanoparticulate imatinib mesylate, or a salt thereof, composition is by spray freezing into liquid (SFL). This technology comprises use of an organic or organoaqueous solution of imatinib mesylate with stabilizers, which is injected into a cryogenic liquid, such as liquid nitrogen. The droplets of the imatinib mesylate solution freeze at a rate sufficient to minimize crystallization and particle growth, thus forming nanostructured imatinib mesylate particles. Depending upon the choice of solvent system and processing conditions, the nanoparticulate imatinib mesylate particles can have varying particle morphology. In the isolation step, the nitrogen and solvent are removed under conditions that avoid agglomeration or ripening of the imatinib mesylate particles.

As a complementary technology to SFL, ultra rapid freezing (URF) may also be used to created equivalent nanostructured imatinib mesylate particles with greatly enhanced surface area. URF comprises an organic or organoaqueous solution of imatinib mesylate with stabilizers onto a cryogenic substrate.

### 5. Emulsion Methodologies to Obtain Nanoparticulate Imatinib Mesylate Compositions

Another method of forming the desired nanoparticulate imatinib mesylate, or a salt composition is by template emulsion. Template emulsion creates nanostructured imatinib mesylate particles with controlled particle size distribution and rapid dissolution performance. The method comprises an oil-in-water emulsion that is prepared, then swelled with a non-aqueous solution comprising the imatinib mesylate and stabilizers. The particle size distribution of the imatinib mesylate particles is a direct result of the size of the emulsion droplets prior to loading with the imatinib mesylate a property which can be controlled and optimized in this process. Furthermore, through selected use of solvents and stabilizers, emulsion stability is achieved with no or suppressed Ostwald ripening. Subsequently, the solvent and water are removed, and the stabilized nanostructured imatinib mesylate particles are recovered. Various imatinib mesylate particles morphologies can be achieved by appropriate control of processing conditions.

Published International Patent Application No. WO 97/144407 to Pace et al., published April 24, 1997, discloses particles of water insoluble biologically active compounds with an average size of 100 nm to 300 nm that are prepared by dissolving the compound in a solution and then spraying the solution into compressed gas, liquid or supercritical fluid in the presence of appropriate surface modifiers.

### D. Methods of Using the Nanoparticulate Imatinib Mesylate Compositions of the Invention

The invention provides a method of increasing the plasma levels of an imatinib mesylate, or a salt thereof, in a subject. Such a method comprises administering to a subject an effective amount of a composition according to the invention comprising nanoparticulate imatinib mesylate compositions.

In one embodiment of the invention, the imatinib mesylate composition, in accordance with standard pharmacokinetic practice, preferably produces a maximum blood plasma concentration profile in less than about 6 hours, less than about 5 hours, less than about 4 hours, less than about 3 hours, less than about 2 hours, less than about 1 hour, or less than about 30 minutes after the initial dose of the composition.

The compositions of the invention are useful in the treatment of chronic myeloid leukemia, gastrointestinal stromal tumors and related diseases. The imatinib mesylate, or a salt thereof, compounds of the invention can be administered to a subject via any conventional means including, but not limited to, orally, rectally, opticly, ocularly, parenterally (*e.g*., intravenous, intramuscular, or subcutaneous, intracisternally, pulmonary, intravaginally, intaperitoneally, locally (*e.g*., powders, ointments or drops), or as a buccal or nasal spray. As used herein, the term "subject" is used to mean an animal, preferably a mammal, including a human or non-human. The terms patient and subject may be used interchangeably.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate imatinib mesylate, or a salt thereof, compositions may also comprise adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents decaying absorption, such as aluminum monostearate and gelatin.

Solid dosage forms for oral administration include, but are not limited to, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to an imatinib mesylate, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil; glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

'Therapeutically effective amount' as used herein with respect to an imatinib mesylate, dosage shall mean that dosage that provides the specific pharmacological response for which an imatinib mesylate is administered in a significant number of subjects in need of such treatment. It is emphasized that 'therapeutically effective amount,' administered to a particular subject in a particular instance will not always be effective in treating the diseases described herein, even though such dosage is deemed a 'therapeutically effective amount' by those skilled in the art. It is to be further understood that imatinib mesylate dosages are, in particular instances, measured as oral dosages, or with reference to drug levels as measured in blood.

One of ordinary skill will appreciate that effective amounts of an imatinib mesylate can be determined empirically and can be employed in pure form or, where such forms exist, in pharmaceutically acceptable salt, ester, or prodrug form. Actual dosage levels of an imatinib mesylate in the nanoparticulate compositions of the invention may be varied to obtain an amount of an imatinib mesylate that is effective to obtain a desired therapeutic response for a particular composition and method of administration. The selected dosage level therefore depends upon the desired therapeutic effect, the route of administration, the potency of the administered imatinib mesylate, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration; and rate of excretion of the agent; the duration of the treatment; drugs used in combination or coincidental with the specific agent; and like factors well know in the medical arts.

The following prophetic example is given to illustrate the present invention.

### Example 1

The purpose of this example was to prepare a composition comprising a nanoparticulate imatinib mesylate or a salt thereof.

An aqueous dispersion of 5% (w/w) imatinib mesylate, combined with one or more surface stabilizers, such as hydroxypropyl cellulose (HPC-SL) and dioctylsulfosuccinate (DOSS), could be milled in a 10 ml chamber of a NanoMill® 0.01 (NanoMill Systems, King of Prussia, PA; see e.g., U.S. Patent No. 6,431,478), along with 500 micron PolyMill® attrition media (Dow Chemical Co.) (e.g., at an 89% media load). In an exemplary process, the mixture could be milled at a speed of 2500 rpms for 60 minutes.

Following milling, the particle size of the milled imatinib mesylate particles can be measured, in deionized distilled water, using a Horiba LA 910 particle size analyzer. For a successful composition, the initial mean and/or D50 milled imatinib mesylate particle size is expected to be less than 2000 nm.

## Claims

1. A stable nanoparticulate composition of imatinib mesylate, or a salt thereof, comprising:
(a) particles of imatinib mesylate, or a salt thereof, having an effective average particle size of less than about 2000 nm; and
(b) at least one surface stabilizer.

2. The composition of claim 1, wherein the imatinib mesylate particles are selected from the group consisting of a crystalline phase, an amorphous phase, a semi-crystalline phase, a semi amorphous phase, and mixtures thereof.

3. The composition of claim 1 or claim 2, wherein the effective average particle size of the imatinib mesylate particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

4. The composition of any one of claims 1 to 3, wherein the composition is formulated:
(a) for administration selected from the group consisting of oral, pulmonary, rectal, colonic, parenteral, intracisternal, intravaginal, intraperitoneal, ocular, optic, local, buccal, nasal, and topical administration;
(b) into a dosage form selected from the group consisting of liquid dispersions, gels, aerosols, ointments, creams, lyophilized formulations, tablets, and capsules;
(c) into a dosage form selected from the group consisting of controlled release formulations, fast melt formulations, delayed release formulations, extended release formulations, pulsatile release formulations, and mixed immediate release and controlled release formulations; or
(d) any combination of (a), (b), and (c).

5. The composition of any one of claims 1 to 4, further comprising one or more pharmaceutically acceptable excipients, carriers, or a combination thereof.

6. The composition of any one of claims 1 to 5, wherein:
(a) imanitib mesylate, or a salt thereof is present in an amount selected from the group consisting of from about 99.5% to about 0.001 %, from about 95% to about 0.1 %, and from about 90% to about 0.5%, by weight, based on the total combined dry weight of imanitib mesylate, or a salt thereof and at least one surface stabilizer, not including other excipients;
(b) the surface stabilizer is present in an amount selected from the group consisting of about 0.5% to about 99.999% by weight, from about 5.0% to about 99.9% by weight, and from about 10% to about 99.5% by weight, based on the total combined dry weight of imanitib mesylate, or a salt thereof and at least one surface stabilizer, not including other excipients; or
(c) a combination thereof.

7. The composition of any one of claims 1 to 6, further comprising at least one primary surface stabilizer and at least one secondary surface stabilizer.

8. The composition of any one of claims 1 to 7, wherein the surface stabilizer is selected from the group consisting of an anionic surface stabilizer, a cationic surface stabilizer, a nonionic surface stabilizer, a zwitterionic surface stabilizer, and an ionic surface stabilizer.

9. The composition of any one of claims 1 to 8, wherein the surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl ß-D-glucopyranoside; n-decyl ß-D-maltopyranoside; n-dodecyl ß-D-glucopyranoside; n-dodecyl ß-D-maltoside; heptanoyl-N-methylglucamide; n-heptyl-p-D-glucopyranoside; n-heptyl ß-D-thioglucoside; n-hexyl ß-D-glucopyranoside; nonanoyl-N-methylglucamide; n-noyl ß-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, random copolymers of vinyl acetate and vinyl pyrrolidone, a cationic polymer, a cationic biopolymer, a cationic polysaccharide, a cationic cellulosic, a cationic alginate, a cationic nonpolymeric compound, a cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, polydiallyldimethylammonium chloride, dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, alkyl pyridinium salts; amines, amine salts, amine oxides, imide azolinium salts, protonated quaternary acrylamides, methylated quaternary polymers, and cationic guar.

10. The composition of any one of claims 1 to 9, wherein the composition is bioadhesive.

11. The composition of any one of claims 1 to 10, wherein the composition does not produce significantly different absorption levels when administered under fed as compared to fasting conditions.

12. The composition comprising imitanib mesylate, or a salt thereof according to any of the preceding claims, wherein upon administration to a human the composition does not produce significantly different absorption levels when administered under fed as compared to fasting conditions.

13. The composition of any one of claims 1 to 12, wherein administration of the composition to a subject in a fasted state is bioequivalent to administration of the composition to a subject in a fed state.

14. The stable nanoparticulate composition of imatinib mesylate, or a salt thereof according to any of the preceding claims, comprising:
(a) particles of imatinib mesylate, or a salt thereof, having an effective average particle size of less than about 2000 nm; and
(b) at least one surface stabilizer,
wherein upon administration to a mammal the composition produces therapeutic results at a dosage which is less than that of a non-nanoparticulate dosage form of the same imatinib mesylate, or salt thereof.

15. The composition of imatinib mesylate, or a salt thereof according to any of the preceding claims, comprising imatinib mesylate or a salt thereof wherein the composition has:
(a) a Cₘₐₓ for imatinib mesylate, or a salt thereof, when assayed in the plasma of a mammalian subject following administration that is greater than the Cₘₐₓ for a non-nanoparticulate formulation of the same imatinib mesylate, or a salt thereof, administered at the same dosage;
(b) an AUC for imatinib mesylate, or a salt thereof, when assayed in the plasma of a mammalian subject following administration that is greater than the AUC for a non-nanoparticulate formulation of the same imatinib mesylate, or a salt thereof, administered at the same dosage;
(c) a Tₘₐₓ for imatinib mesylate, or a salt thereof, when assayed in the plasma of a mammalian subject following administration that is less than the Tₘₐₓ for a non-nanoparticulate formulation of the same imatinib mesylate, or a salt thereof, administered at the same dosage; or
(d) any combination of (a), (b), and (c).

16. The composition of any one of claims 1 to 15, additionally comprising one or more active agents useful for the treatment of chronic myeloid leukemia, gastrointestinal stromal tumors and related diseases.

17. The composition of claim 16, wherein the active agent is selected from a group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, and anti-androgens.

18. A composition of any one of claims 1 to 17 for use in therapy.

19. A composition of any one of claims 1 to 17 for use in the treatment of chronic myeloid leukemia, gastrointestinal stromal tumors or related diseases.

20. Use of a composition of any one of claims 1 to 17 for the manufacture of a medicament for treatment of chronic myeloid leukemia, gastrointestinal stromal tumors or related diseases.

21. A method for preparing imatinib mesylate, or a salt thereof, comprising contacting particles of imatinib mesylate, or a salt thereof with at least one surface stabilizer for a time and under conditions sufficient to provide a nanoparticulate imatinib mesylate composition having an effective average particle size of less than about 2000 nm.

22. The method of claim 21, wherein the contacting comprises grinding, wet grinding, homogenization, freezing, template emulsion, precipitation, or a combination thereof.

## Patentansprüche

1. Stabile Nanopartikelzusammensetzung von Imatinibmesylat oder einem Salz davon, umfassend:
(a) Partikel von Imatinibmesylat oder einem Salz davon mit einer effektiven mittleren Partikelgröße von weniger als etwa 2000 nm; und
(b) mindestens einen Oberflächenstabilisator.

2. Zusammensetzung nach Anspruch 1, wobei die Imatinibmesylatpartikel aus der Gruppe ausgewählt sind, bestehend aus einer kristallinen Phase, einer amorphen Phase, einer halbkristallinen Phase, einer halbamorphen Phase und Mischungen davon.

3. Zusammensetzung nach Anspruch 1 oder Anspruch 2, wobei die effektive mittlere Partikelgröße der Imatinibmesylatpartikel aus der Gruppe ausgewählt ist, bestehend aus weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm.

4. Zusammensetzung nach einem der Ansprüche 1 bis 3, wobei die Zusammensetzung wie folgt formuliert wird:
(a) für eine Verabreichung ausgewählt aus der Gruppe bestehend aus oraler, pulmonaler, rektaler, enteraler, parenteraler, intracisternaler, intravaginaler, intraperitonealer, okularer, opthalmischer, lokaler, buccaler, nasaler und topischer Verabreichung;
(b) in eine Darreichungsform, ausgewählt aus der Gruppe bestehend aus flüssigen Dispersionen, Gelen, Aerosolen, Salben, Cremes, lyophilisierten Formulierungen, Tabletten und Kapseln;
(c) in eine Darreichungsform, ausgewählt aus der Gruppe bestehend aus Formulierungen mit kontrollierter Freisetzung, schnell zergehenden Formulierungen, Formulierungen mit verzögerter Freisetzung, Formulierungen mit verlängerter Freisetzung, Formulierungen mit pulsierender Freisetzung und Formulierungen mit gemischter sofortiger und kontrollierter Freisetzung; oder
(d) eine beliebige Kombination aus (a), (b) und (c).

5. Zusammensetzung nach einem der Ansprüche 1 bis 4, des Weiteren enthaltend einen oder mehrere pharmazeutisch zulässige Hilfsstoffe, Arzneimittelträger oder eine Kombination davon.

6. Zusammensetzung nach einem der Ansprüche 1 bis 5, wobei:
(a) Imatinibmesylat oder ein Salz davon in einer Menge vorliegt, ausgewählt aus der Gruppe bestehend aus von etwa 99,5 Gew.-% bis etwa 0,001 Gew.-%, von etwa 95 Gew.-% bis etwa 0,1 Gew.-% und von etwa 90 Gew.-% bis etwa 0,5 Gew.-%, basierend auf dem gesamten, kombinierten Trockengewicht von Imatinibmesylat oder einem Salz davon und mindestens einem Oberflächenstabilisator, wobei weitere Hilfsstoffe nicht eingeschlossen sind;
(b) der Oberflächenstabilisator in einer Menge vorliegt, ausgewählt aus der Gruppe bestehend aus von etwa 0,5 Gew.-% bis etwa 99,999 Gew.-%, von etwa 5,0 Gew.-% bis etwa 99,9 Gew.-% und von etwa 10 Gew.-% bis etwa 99,5 Gew.-%, basierend auf dem gesamten, kombinierten Trockengewicht von Imatinibmesylat oder einem Salz davon und mindestens einem Oberflächenstabilisator, wobei weitere Hilfsstoffe nicht eingeschlossen sind; oder
(c) eine Kombination davon.

7. Zusammensetzung nach einem der Ansprüche 1 bis 6, des Weiteren enthaltend mindestens einen primären Oberflächenstabilisator und mindestens einen sekundären Oberflächenstabilisator.

8. Zusammensetzung nach einem der Ansprüche 1 bis 7, wobei der Oberflächenstabilisator aus der Gruppe ausgewählt ist, bestehend aus einem anionischen Oberflächenstabilisator, einem kationischen Oberflächenstabilisator, einem nicht-ionischen Oberflächenstabilisator, einem zwitterionischen Oberflächenstabilisator und einem ionischen Oberflächenstabilisator.

9. Zusammensetzung nach einem der Ansprüche 1 bis 8, wobei der Oberflächenstabilisator aus der Gruppe ausgewählt ist, bestehend aus Cetylpyridiniumchlorid, Gelatine, Casein, Phosphatiden, Dextran, Glycerin, Gummi arabicum, Cholesterin, Tragant, Stearinsäure, Benzalkoniumchlorid, Calciumstearat, Glycerinmonostearat, Cetostearylalkohol, emulgierendem Cetomacrogol-Wachs, Sorbitanestern, Polyoxyethylenalkylethern, Polyoxyethylen-Ricinusöl-Derivaten, Polyoxyethylensorbitanfettsäureestern, Polyethylenglykolen, Dodecyltrimethylammoniumbromid, Polyoxyethylenstearaten, kolloidalem Siliciumdioxid, Phosphaten, Natriumdodecylsulfat, Calcium-Carboxymethylcellulose, Hydroxypropylcellulosen, Hypromellose, NatriumCarboxymethylcellulose, Methylcellulose, Hydroxyethylcellulose, Hypromellosephthalat, nichtkristalliner Cellulose, Magnesiumaluminiumsilikat, Triethanolamin, Polyvinylalkohol, Polyvinylpyrrolidon, 4-(1,1,3,3-Tetramethylbutyl)phenol-Polymer mit Ethylenoxid und Formaldehyd, Poloxameren; Poloxaminen, einem geladenen Phospholipid, Dioctylsulfosuccinat, Dialkylestern von Natriumsulfobernsteinsäure, Natriumlaurylsulfat, Alkylarylpolyethersulfonaten, Mischungen von Saccharosestearat und Saccharosedistearat, p-Isononylphenoxypoly(glycidol), Decanoyl-N-methylglucamid; n-Decyl-β-D-glucopyranosid; n-Decyl-β-D-maltopyranosid; n-Dodecyl-β-D-glucopyranosid; n-Dodecyl-β-D-maltosid; Heptanoyl-N-methylglucamid; n-Heptyl-β-D-glucopyranosid; n-Heptyl-β-D-thioglucosid; n-Hexyl-β-D-glucopyranosid; Nonanoyl-N-methylglucamid; n-Nonyl-β-D-glucopyranosid; Octanoyl-N-methylglucamid; n-Octyl-β-D-glucopyranosid; Octyl-β-D-thioglucopyranosid; Lysozym, PEG-Phospholipid, PEG-Cholesterin, PEG-Cholesterinderivat, PEG-Vitamin A, PEG-Vitamin E, statistischen Copolymeren von Vinylacetat und Vinylpyrrolidon, einem kationischen Polymer, einem kationischen Biopolymer, einem kationischen Polysaccharid, einer kationischen Cellulose, einem kationischen Alginat, einer kationischen, nicht polymeren Verbindung, einem kationischen Phospholipid, kationischen Lipiden, Polymethylmethacrylat-trimethylammoniumbromid, Sulfoniumverbindungen, Polyvinylpyrrolidon-2-dimethylaminoethylmethacrylat-dimethylsulfat, Hexadecyltrimethylammoniumbromid, Phosphoniumverbindungen, quartären Ammoniumverbindungen, Benzyl-di-(2-chlorethyl)ethylammoniumbromid, Kokosnuss-Trimethylammoniumchlorid, Kokosnuss-Trimethylammoniumbromid, Kokosnuss-Methyldihydroxyethylammoniumchlorid, Kokosnuss-Methyldihydroxyethylammoniumbromid, Decyltriethylammoniumchlorid, Decyldimethylhydroxyethylammoniumchlorid, Decyldimethylhydroxyethylammoniumchloridbromid, C₁₂-₁₅-Dimethylhydroxyethylammoniumchlorid, C₁₂₋₁₅-Dimethylhydroxyethylammoniumchloridbromid, Kokosnuss-Dimethylhydroxyethylammoniumchlorid, Kokosnuss-Dimethylhydroxyethylammoniumbromid, Myristyltrimethylammoniummethylsulfat, Lauryldimethylbenzylammoniumchlorid, Lauryldimethylbenzylammoniumbromid, Lauryldimethyl-(ethenoxy)₄-ammoniumchlorid, Lauryldimethyl-(ethenoxy)₄-ammoniumbromid, N-Alkyl-(C₁₂-₁₈)-dimethylbenzylammoniumchlorid, N-Alkyl-(C₁₄₋₁₈)-dimethylbenzylammoniumchlorid, N-Tetradecylidmethylbenzylammoniumchlorid-Monohydrat, Dimethyldidecylammoniumchlorid, N-Alkyl- und (C₁₂₋₁₄)-dimethyl-1-napthylmethylammoniumchlorid, Trimethylammoniumhalogenid, Alkyl-trimethylammoniumsalzen, Dialkyl-dimethylammoniumsalze, Lauryltrimethylammoniumchlorid, ethoxyliertem Alkylamidoalkyldialkylammoniumsalz, einem ethoxylierten Trialkylammoniumsalz, Dialkylbenzoldialkylammoniumchlorid, N-Decyldimethylammoniumchlorid, N-Tetradecyldimethylbenzylammoniumchlorid-Monohydrat, N-Alkyl-(C₁₂₋₁₄)-dimethyl-1-naphthylmethylammoniumchlorid, Dodecyldimethylbenzylammoniumchlorid, Dialkylbenzolalkylammoniumchlorid, Lauryltrimethylammoniumchlorid, Alkylbenzylmethylammoniumchlorid, Alkylbenzyldimethylammoniumbromid, C₁₂-Trimethylammoniumbromiden, C₁₅-Trimethylammoniumbromiden, C₁₇-Trimethylammoniumbromiden, Dodecylbenzyltriethylammoniumchlorid, Poly-diallyldimethylammoniumchlorid, Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrioctylammoniumchlorid, Tetrabutylammoniumbromid, Benzyltrimethylammoniumbromid, Cholinestern, Benzalkoniumchlorid, Stearalkoniumchlorid-Verbindungen, Cetylpyridiniumbromid, Cetylpyridiniumchlorid, Halogenidsalzen von quarternisierten Polyoxyethylalkylaminen, Alkylpyridiniumsalzen; Aminen, Aminsalzen, Aminoxiden, Imidazoliniumsalzen, protonierten quartären Acrylamiden, methylierten quartären Polymeren und kationischem Guar.

10. Zusammensetzung nach einem der Ansprüche 1 bis 9, wobei die Zusammensetzung bioadhäsiv ist.

11. Zusammensetzung nach einem der Ansprüche 1 bis 10, wobei die Zusammensetzung keine signifikant unterschiedlichen Absorptionsspiegel erzeugt, wenn sie nüchtern oder nach Nahrungsaufnahme verabreicht wird.

12. Zusammensetzung, umfassend Imatinibmesylat oder ein Salz davon nach einem der vorhergehenden Ansprüche, wobei die Zusammensetzung bei Verabreichung an einen Menschen keine signifikant unterschiedlichen Absorptionsspiegel erzeugt, wenn sie nüchtern oder nach Nahrungsaufnahme verabreicht wird.

13. Zusammensetzung nach einem der Ansprüche 1 bis 12, wobei eine Verabreichung der Zusammensetzung an einen Patienten im nüchternen Zustand bioäquivalent zur Verabreichung der Zusammensetzung an einen Patienten nach Nahrungsaufnahme ist.

14. Stabile Nanopartikelzusammensetzung von Imatinibmesylat oder einem Salz davon nach einem der vorhergehenden Ansprüche, umfassend:
(a) Partikel von Imatinibmesylat oder einem Salz davon mit einer effektiven mittleren Partikelgröße von weniger als etwa 2000 nm; und
(b) mindestens ein Oberflächenstabilisator,
wobei die Zusammensetzung bei Verabreichung an ein Säugetier therapeutische Ergebnisse bei einer Dosierung erzeugt, die geringer ist als die einer nicht-nanopartikulären Darreichungsform des gleichen Imatinibmesylats oder eines Salzes davon.

15. Zusammensetzung von Imatinibmesylat oder einem Salz davon nach einem der vorhergehenden Ansprüche, umfassend Imatinibmesylat oder ein Salz davon, wobei die Zusammensetzung folgendes aufweist:
(a) ein Cₘₐₓ für Imatinibmesylat oder einem Salz davon, der bei Untersuchung im Plasma eines Säugetierpatienten nach Verabreichung größer ist als der Cₘₐₓ für eine nicht-nanopartikuläre Formulierung des gleichen Imatinibmesylats oder eines Salzes davon, bei Verabreichung in der gleichen Dosierung;
(b) ein AUC für Imatinibmesylat oder einem Salz davon, der bei Untersuchung im Plasma eines Säugetierpatienten nach Verabreichung größer ist als der AUC für eine nicht-nanopartikuläre Formulierung des gleichen Imatinibmesylats oder eines Salzes davon, bei Verabreichung in der gleichen Dosierung;
(c) ein Tₘₐₓ für Imatinibmesylat oder einem Salz davon, der bei Untersuchung im Plasma eines Säugetierpatienten nach Verabreichung geringer ist als der Tₘₐₓ für eine nicht-nanopartikuläre Formulierung des gleichen Imatinibmesylats oder eines Salzes davon, bei Verabreichung in der gleichen Dosierung; oder
(d) eine beliebige Kombination aus (a), (b) und (c).

16. Zusammensetzung nach einem der Ansprüche 1 bis 15, zusätzlich umfassend ein oder mehrere Wirkstoffe, die für die Behandlung von chronischer myeloischer Leukämie, gastrointestinalen Stromatumoren und verwandten Erkrankungen von Nutzen sind.

17. Zusammensetzung nach Anspruch 16, wobei der Wirkstoff aus der Gruppe ausgewählt ist, bestehend aus Mitosehemmern, alkylierenden Mitteln, Antimetaboliten, interkalierenden Antibiotika, Wachstumsfaktorhemmern, Zellzyklushemmern, Enzymen, Topoisomerasehemmern, BRM-Substanzen (Biological Response Modifier), Antihormonen und Antiandrogenen.

18. Zusammensetzung nach einem der Ansprüche 1 bis 17 für die Verwendung in der Therapie.

19. Zusammensetzung nach einem der Ansprüche 1 bis 17 für die Behandlung von chronischer myeloischer Leukämie, gastrointestinalen Stromatumoren oder verwandten Erkrankungen.

20. Verwendung einer Zusammensetzung nach einem der Ansprüche 1 bis 17 zur Herstellung eines Arzneimittels für die Behandlung von chronischer myeloischer Leukämie, gastrointestinalen Stromatumoren oder verwandten Erkrankungen.

21. Verfahren zur Herstellung von Imatinibmesylat oder einem Salz davon, umfassend das In-Kontakt-Bringen von Partikeln aus Imatinibmesylat oder einem Salz davon mit mindestens einem Oberflächenstabilisator über einen Zeitraum und unter Bedingungen, die ausreichen, um eine nanopartikuläre Imatinibmesylat-Zusammensetzung mit einer effektiven mittleren Partikelgröße von weniger als 2000 nm zu ergeben.

22. Verfahren nach Anspruch 21, wobei das In-Kontakt-Bringen Mahlen, Nassmahlen, Homogenisieren, Einfrieren, Templatemulsion, Ausfällen oder eine Kombination davon umfasst.

## Revendications

1. Composition de nanoparticules stable de mésylate d'imatinib ou sel de ce dernier, comprenant :
(a) des particules de mésylate d'imatinib ou un sel de ce dernier présentant une taille de particule moyenne effective de moins d'environ 2000 nm ; et
(b) au moins un stabilisateur de surface.

2. Composition selon la revendication 1, dans laquelle les particules de mésylate d'imatinib sont sélectionnées dans le groupe constitué d'une phase cristalline, d'une phase amorphe, d'une phase semi-cristalline, d'une phase semi-amorphe et de mélange de ces dernières.

3. Composition selon la revendication 1 ou la revendication 2, dans laquelle la taille de particule moyenne effective des particules de mésylate d'imatinib est sélectionnée dans le groupe constitué de moins d'environ 1900 nm, moins d'environ 1800 nm, moins d'environ 1700 nm, moins d'environ 1600 nm, moins d'environ 1500 nm, moins d'environ 1400 nm, moins d'environ 1300 nm, moins d'environ 1200 nm, moins d'environ 1100 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 100 nm, moins d'environ 75 nm, et moins d'environ 50 nm.

4. Composition selon l'une quelconque des revendications 1 à 3, dans laquelle la composition est formulée :
(a) pour une administration sélectionnée dans le groupe constitué d'administration orale, pulmonaire, rectale, colonique, parentérale, intra-cistemale, intra-vaginale, intra-péritonéale, oculaire, optique, locale, buccale, nasale, et topique ;
(b) en une forme de dosage sélectionnée dans le groupe constitué de dispersions liquides, gels, aérosols, onguents, crèmes, formulations lyophilisées, comprimés et capsules ;
(c) en une forme de dosage sélectionnée dans le groupe constitué de formulations à libération contrôlée, formulations à fusion rapide, formulations à libération retardées, formulations à libération étendue, formulations à libération pulsatile, et formulations mixtes à libération immédiate et à libération contrôlée ; ou
(d) toute combinaison of (a), (b) et (c).

5. Composition selon l'une quelconque des revendications 1 à 4, comprenant en outre un ou plusieurs excipients, porteurs ou une combinaison de ces derniers pharmaceutiquement acceptables.

6. Composition selon l'une quelconque des revendications 1 à 5, dans laquelle :
(a) du mésylate d'imatinib, ou un sel de ce dernier existe en une concentration sélectionnée dans le groupe constitué d'environ 99,5% à environ 0,001%, d'environ 95% à environ 0,1%, et d'environ 90% à environ 0,5%, en poids, sur la base du poids à sec total combiné de mésylate d'imatinib, ou un sel de ce dernier et au moins un stabilisateur de surface, autres excipients non compris ;
(b) le stabilisateur de surface existe en une concentration sélectionnée dans le groupe constitué d'environ 0,5% à environ 99,999% en poids, d'environ 5,0% à environ 99,9% en poids, et d'environ 10% à environ 99,5% en poids, sur la base du poids à sec total combiné de mésylate d'imatinib, ou un sel de ce dernier et au moins un stabilisateur de surface, autres excipients non compris ; ou
(c) une combinaison de ces derniers.

7. Composition selon l'une quelconque des revendications 1 à 6, comprenant en outre au moins un stabilisateur de surface primaire et au moins un stabilisateur de surface secondaire.

8. Composition selon l'une quelconque des revendications 1 à 7, dans laquelle le stabilisateur de surface est sélectionné dans le groupe constitué d'un stabilisateur de surface anionique, un stabilisateur de surface cationique, un stabilisateur de surface non ionique, un stabilisateur de surface zwitterionique et un stabilisateur de surface ionique.

9. Composition selon l'une quelconque des revendications 1 à 8, dans laquelle le stabilisateur de surface est sélectionné dans le groupe constitué de chlorure de pyridinium cétylique, gélatine, caséine, phosphatides, dextrane, glycérol, gomme d'acacias, cholestérol, gomme adragante, acide stéarique, chlorure de benzalkonium, stéarate de calcium, monostéarate de glycérol, alcool cétostéarylique, wax émulsifiant de cétomacrogol, esters de sorbitane, esters alkyliques de polyoxyéthylène, dérivés d'huile de ricin au polyoxyéthylène, esters d'acides gras de sorbitane au polyoxyéthylène, glycols polyéthyléniques, bromure d'ammonium triméthylique dodécylique, stéarates polyoxyéthyléniques, dioxyde de silicium colloïdal, phosphates, dodécylsulfate de sodium, carboxyméthylcellulose calcique, celluloses hydroxypropyliques, hypromelloses, carboxyméthylcellulose sodique, méthylcellulose, hydroxyéthylcellulose, phthalate d'hypromellose, cellulose non cristalline, silicate aluminomagnésien, triéthanolamine, alcool polyvinylique, polyvinylpyrrolidone, polymère 4-(1,1,3,3-tétraméthylbutyl)-phénol avec oxyde d'éthylène et formaldéhyde, poloxamères ; poloxamines, un phospholipide chargé, dioctylsulfosuccinate, des dialkylesters d'acide sulfosuccinique de sodium, sulfate laurylique de sodium, alkylarylsulfonates de polyéther, mélanges de stéarate de sucrose et de distéarate de sucrose, p-isononylphénoxypoly- (glycidol), décanoyl-N-méthylglucamide ; n-décyl β-D-glucopyranoside ; n-décyl β-D- maltopyranoside ; n-dodécyl β-D-glucopyranoside ; n-dodécyl β-D-maltoside ; heptanoyl-N-méthylglucamide ; n-heptyl-β-D-glucopyranoside ; n-heptyl β-D-thioglucoside ; n-hexyl β-D- glucopyranoside ; nonanoyl-N-méthylglucamide ; n-noyl β-D-glucopyranoside ; octanoyl-N-méthylglucamide ; n-octyl-β-D-glucopyranoside ; octyl β-D-thioglucopyranoside ; lysozyme , phospholipide PEG, cholestérol PEG, dérivé de cholestérol PEG , vitamine A PEG, vitamine E PEG, copolymères aléatoires d'acétate de vinyle et de vinylpyrrolidone, un polymère cationique, un biopolymère cationique, un polysaccharide cationique, une cellulose cationique, une alginate cationique, un compose non polymérique cationique, un phospholipide cationique, des lipides cationiques, bromure de polyméthylméthacrylate-triméthylammonium, composés de sulfonium, sulfate de polyvinylpyrrolidone-2-diméthylaminoéthyl méthacrylate-diméthyl, bromure d'hexadécyltriméthylammonium, composés de phosphonium, composés d'ammonium quaternaires, bromure de benzyl- di(2-chloroéthyl)éthylammonium, chlorure de triméthyl ammonium de coco, bromure de triméthyl ammonium de coco, chlorure de méthyl dihydroxyéthyl ammonium de coco, bromure de méthyl dihydroxyéthyl ammonium de coco, chlorure de décyl triéthyl ammonium, chlorure de décyl diméthyl hydroxyéthyl ammonium, bromure de chlorure de décyl diméthyl hydroxyéthyl ammonium, chlorure de C₁₂₋₁₅diméthyl hydroxyéthyl ammonium, bromure de chlorure de C₁₂₋₁₅diméthyl hydroxyéthyl ammonium, chlorure de diméthyl hydroxyéthyl ammonium de coco, bromure de diméthyl hydroxyéthyl ammonium de coco, méthylsulphate de myristyl triméthyl ammonium, chlorure de lauryl diméthyl benzyl ammonium, bromure de lauryl diméthyl benzyl ammonium, chlorure de lauryl diméthyl (éthénoxy)₄ ammonium, bromure de lauryl diméthyl (éthénoxy)₄ ammonium, chlorure de N-alkyl (C₁₂₋₁₈)diméthylbenzyl ammonium, chlorure de N-alkyl (C₁₄₋₁₈)diméthyl-benzyl ammonium, monohydrate de chlorure de N-tetradécylidméthylbenzyl ammonium, chlorure de diméthyl didécyl ammonium, chlorure de N-alkyl et (C₁₂₋₁₄) diméthyl 1-napthylméthyl ammonium, halogénure de triméthylammonium, sels d'alkyl-triméthylammonium, sels de dialkyl-diméthylammonium, chlorure de lauryl triméthyl ammonium, sel d'alkyamidoalkyldialkylammonium éthoxylé, un sel de trialkyl ammonium éthoxylé, chlorure de dialkylbenzène dialkylammonium, chlorure de N-didécyldiméthyl ammonium, N-tetradécyldiméthylbenzyl ammonium, chlorure monohydraté, chlorure de N-alkyl(C₁₂₋₁₄) diméthyl 1-naphthylméthyl ammonium, chlorure de dodécyldiméthylbenzyl ammonium, chlorure de dialkyl benzènealkyl ammonium, chlorure de lauryl triméthyl ammonium, chlorure d'alkylbenzyl méthyl ammonium, bromure d'alkyl benzyl diméthyl ammonium, bromures de C₁₂ triméthyl ammonium, bromures de C₁₅ triméthyl ammonium, bromures de C₁₇ triméthyl ammonium, chlorure de dodécylbenzyl triéthyl ammonium, chlorure de poly- diallyldiméthylammonium, chlorures de diméthyl ammonium, halogénures d'alkyldiméthylammonium, chlorure de tricétyl méthyl ammonium, bromure de décyltriméthylammonium, bromure de dodécyltriéthylammonium, bromure de tetradécyltriméthylammonium, chlorure de méthyl trioctylammonium, bromure de tétrabutylammonium, bromure de benzyl triméthylammonium, cholinesters, chlorure de benzalkonium, composés de chlorure de stéaralkonium, bromure de cétyl pyridinium, chlorure de cétyl pyridinium, sels halogénures de polyoxyéthylalkylamines quaternisés, sels d'alkyl pyridinium ; amines, sels d'amines, oxydes d'amines, sels imide azolinium, acrylamides quaternaires protonés, polymères quaternaires méthylés, et guar cationique.

10. Composition selon l'une quelconque des revendications 1 à 9, dans laquelle la composition est bio-adhésive.

11. Composition selon l'une quelconque des revendications 1 à 10, dans laquelle la composition ne produit pas des niveaux d'absorption considérablement différents lorsqu'elle est administrée avec la nourriture en comparaison avec les conditions à jeun.

12. Composition comprenant du mésylate d'imatinib ou un sel de ce dernier selon l'une quelconque des revendications précédentes, dans laquelle la composition, en cas d'administration à un être humain, ne produit pas des niveaux d'absorption considérablement différents lorsqu'elle est administrée avec la nourriture en comparaison avec les conditions à jeun.

13. Composition selon l'une quelconque des revendications 1 à 12, dans laquelle l'administration de la composition à un sujet à jeun est bio-équivalent à l'administration de la composition à un sujet avec de la nourriture.

14. Composition de nanoparticules stable de mésylate d'imatinib ou sel de ce dernier selon l'une quelconque des revendications précédentes, comprenant :
(a) des particules de mésylate d'imatinib ou un sel de ce dernier présentant une taille de particule moyenne effective de moins d'environ 2000 nm ; et
(b) au moins un stabilisateur de surface,
dans laquelle, en cas d'administration à un mammifère, la composition produit des résultats thérapeutiques à un dosage inférieur à celui d'une forme de dosage sans nanoparticules du même mésylate d'imatinib ou sel de ce dernier.

15. Composition de mésylate d'imatinib ou sel de ce dernier selon l'une quelconque des revendications précédentes, comprenant du mésylate d'imatinib ou un sel de ce dernier dans lequel la composition présente :
(a) un Cₘₐₓ pour mésylate d'imatinib ou un sel de ce dernier lorsqu'elle est essayée dans le plasma d'un sujet mammifère après une administration plus grande que le Cₘₐₓ pour une formulation sans nanoparticules du même mésylate d'imatinib ou sel de ce dernier lorsqu'elle administrée avec le même dosage ;
(b) un AUC pour mésylate d'imatinib ou un sel de ce dernier lorsqu'elle est essayée dans le plasma d'un sujet mammifère après une administration plus grande que le AUC pour une formulation sans nanoparticules du même mésylate d'imatinib ou sel de ce dernier lorsqu'elle administrée avec le même dosage ;
(c) un Tₘₐₓ pour mésylate d'imatinib ou un sel de ce dernier lorsqu'elle est essayée dans le plasma d'un sujet mammifère après une administration plus petite que le Tₘₐₓ pour une formulation sans nanoparticules du même mésylate d'imatinib ou sel de ce dernier lorsqu'elle administrée avec le même dosage ; ou
(d) toute combinaison of (a), (b) et (c).

16. Composition selon l'une quelconque des revendications 1 à 15, comprenant en outre un ou plusieurs agents actifs utiles pour le traitement de la leucémie myéloïde chronique, de tumeurs gastro-intestinales internes et maladies apparentées.

17. Composition selon la revendication 16, dans laquelle l'agent actif est sélectionné dans le groupe constitué d'inhibiteurs mitotiques, d'agents alkylants, d'anti-métabolites, d'antibiotiques intercalaires, d'inhibiteurs de facteur de croissance, d'inhibiteurs de cycle cellulaire, d'enzymes, d'inhibiteurs de topoisomérase, de modificateurs de réponse biologique, d'antihormones et d'anti-androgènes.

18. Composition selon l'une quelconque des revendications 1 à 17 pour utilisation dans le cadre d'une thérapie.

19. Composition selon l'une quelconque des revendications 1 à 17 pour utilisation dans le cadre du traitement de la leucémie myéloïde chronique, de tumeurs gastro-intestinales internes ou de maladies apparentées.

20. Utilisation d'une composition selon l'une quelconque des revendications 1 à 17 pour la fabrication d'un médicament pour le traitement de la leucémie myéloïde chronique, de tumeurs gastro-intestinales internes ou de maladies apparentées.

21. Méthode pour la préparation de mésylate d'imatinib ou d'un sel de ce dernier, comprenant le contact avec des particules de mésylate d'imatinib ou d'un sel de ce dernier avec au moins un stabilisateur de surface pendant une durée et sous des conditions suffisantes pour fournir une composition de mésylate d'imatinib à nanoparticules présentant une taille de particule moyenne effective de moins d'environ 2000 nm.

22. Méthode selon la revendication 21, dans laquelle le contact comprend le meulage, le meulage à eau, l'homogénéisation, le gel, l'émulsion de matrice, la précipitation ou une combinaison de ces derniers.
